# EUROPEAN PATENT SPECIFICATION

(11) **EP 1 906 177 B1**
(45) Date of publication and mention of the grant of the patent: **13.01.2010**
(21) Application number: 06291523.6
(22) Date of filing: 27.09.2006
(51) Int. Cl.: G01N 27/327, C12Q 1/00

(54) **Microsensor for detection of d-amino-acids**
Mikrosensor zur Bestimmung von D-Aminosäuren
Microcapteur pour la détection d'acides aminés D

(43) Date of publication of application: 02.04.2008
(73) Proprietor: Centre National de la Recherche Scientifique (CNRS), 75794 Paris Cedex 16 (FR); Universite Claude Bernard de Lyon 1, 69622 Villeurbanne Cedex (FR); Université Paris XI, 91405 Orsay Cedex (FR); Université d'Insubria, 21100 Varese (IT)
(72) Inventor: Marinesco, Stéphane, 91190 Gif-sur-Yvette (FR); Pernot, Pierre, 91400 Orsay (FR); Mothet, Jean-Pierre, 75020 Paris (FR); Cespuglio, Raymond, 69390 Millery (FR); Schuvailo, Oleg, 69009 Lyon (FR); Soldatkin, Alexey, Kiev 04211 (UA); Pollegioni, Loredano, 20152 Milano (IT); Pilone, Mirella, 20121 Milano (IT)
(74) Representative: Novagraaf IP

(56) References cited:
- EP-A- 1 542 017
- US-A1- 5 938 903
- JOHANSSON E ET AL: "STUDY OF A REAGENT- NAD MEDIATORLESS BIOSENSOR FOR D-AMINO ACIDS BASED ON CO-IMMOBILIZED D-AMINO ACID OXIDASE AND PEROXIDASE IN CARBON PASTE ELECTRODES" JOURNAL OF BIOMATERIALS APPLICATIONS, TECHNOMIC, LANCASTER, PA, US, vol. 8, October 1993 (1993-10), pages 146-173, XP008075336 ISSN: 0885-3282
- QIJIN CHI ET AL: "AMPEROMETRIC BIOSENSORS BASED ON THE IMMOBILIZATION OF OXIDASES IN A PRUSSIAN BLUE FILM BY ELECTROCHEMICAL CODEPOSITION" ANALYTICA CHIMICA ACTA, ELSEVIER, AMSTERDAM, NL, vol. 310, 1995, pages 429-436, XP008075382 ISSN: 0003-2670
- KURITA R ET AL: "Microfluidic device integrated with pre-reactor and dual enzyme-modified microelectrodes for monitoring in vivo glucose and lactate" SENSORS AND ACTUATORS B, ELSEVIER SEQUOIA S.A., LAUSANNE, CH, vol. 87, no. 2, 10 December 2002 (2002-12-10), pages 296-303, XP004391053 ISSN: 0925-4005
- DOMINGUEZ R ET AL: "CHIRAL ANALYSIS OF AMINO ACIDS USING ELECTROCHEMICAL COMPOSITE BIENZYME BIOSENSORS" ANALYTICAL BIOCHEMISTRY, ACADEMIC PRESS, NEW YORK, NY, US, vol. 298, no. 2, 15 October 2001 (2001-10-15), pages 275-282, XP008075363 ISSN: 0003-2697
- YABUKI S ET AL: "Preparation of d-amino acid oxidase-immobilized polyion complex membranes" SENSORS AND ACTUATORS B, ELSEVIER SEQUOIA S.A., LAUSANNE, CH, vol. 76, no. 1-3, 1 June 2001 (2001-06-01), pages 142-146, XP004241107 ISSN: 0925-4005
- PERNOT, P. ET AL.: ANAL. CHEM., vol. 80, no. 5, 2008, pages 1589-1597,

## Description

The instant invention relates to the field of microsensors or sensing microdevices.
More precisely the invention concerns a microsensor or microelectrode for measurement of D-serine and an electrochemical method for detecting and/or measuring D-amino acid, in particular D-serine, more specifically *in vitro, ex vivo* and/or *in vivo*.

There is specially a need for D-amino-acid sensors that can be used for in vivo measurement. For example, for D-serine, which has been recently shown to be present in the Central Nervous System (CNS), in the cortex, hippocampus or developing cerebellum.
This D-amino acid has been recently implicated in several pathologies such as Schizophrenia, Alzheimer disease, chronic pain or cerebral ischemia. There is thus a need for pharmacological agents able to interfere with synthesis, release, catabolism and/or uptake system of D-serine in the CNS, as well as for reliable methods for detecting D-serine *in vivo* and *in vitro.*
A known method to measure extracellular concentration of D-serine is microdialysis. This method is heavy, expensive and difficult to employ. Moreover, it involves the use of relatively large probes, which may provoke lesions that can compromise the measurement.
Furthermore the principle of this method by itself, comprising the steps of dialysing the extracellular medium, collecting and analysing its content, can induce a perturbation in the physiological functionality of the sample due to the circulation of exogenous extracellular fluid, which may change the local concentration of D-serine as well as of other small metabolites.

Electrochemical methods to detect D-serine are also known, for example in food industry. These methods use sensors having a millimetric or centimetric size, which is generally not compatible with an *in vivo* use. E.g. Johansson, E. et al. J. Biomat. Appl. 1993, Vol. 8, pages 146-173.

The field of microsensors is of increasing interest. There is thus a general need for reliable, cheap, small, precise, selective and/or versatile sensors and more particularly for sensors which can be used *in vivo* and/or allowing to measure in real time the changes in the concentration of a compound.

As discussed above, there is a special need for a device for detecting D-amino-acids, and in particular D-serine, *in vivo* and/or allowing to measure in real time its concentration, for example in order to develop a method to find pharmacological agents able to interfere with synthesis, release and/or elimination of D-amino-acids, and more specifically D-serine in the central nervous system (CNS).

Following a first aspect, the subject matter of the present invention is a microelectrode, for measuring and/or detecting the concentration of a D-Serine, said microelectrode comprising:
- means for oxidation of said D-Serine thereby generating hydrogen peroxide, said means being a D-amino-acid oxydase from a yeast or a microorganism chosen from the group comprising *Rhodotorula gracilis DAAO (RgDAAO), Trigonopsis variabilis DAAO, V. luteoalbum DAAO, F. oxysporum DAAO,*
- means for optimising the detection of said hydrogen peroxide, said means being means for the catalysis of hydrogen peroxide oxidation, and
- means for reducing interferences, said means being limiting oxidation of compounds other than hydrogen peroxide.

The microelectrode of the invention may have a detection limit of 300 nM or less of the D-serine.
The microelectrode presents a good selectivity. For example, solutions of serotonin, dopamine, L-serine and glycine at 10 µM (concentrations much higher than the physiological concentrations of these molecules), do not generate signals bigger than 5% of the ones detected in the same concentration of D-serine.

The Figure 1 shows an example experiment where 200 nM of hydrogen peroxide was injected in the recording chamber. The injection produces a step in the oxidation current at the working electrode.
The Figure 2 shows the calibration of the microbiosensor of the invention in increasing concentrations of hydrogen peroxide. The microelectrode's response is linear with concentration between 10 nM and at least 10 µM of hydrogen peroxide.

By "microelectrode" in the instant invention is meant a small size electrode, in particular an electrode having a mean diameter of less than 1 mm, specially of less than 500 µm, more particularly of less than 250 µm, especially of less than 150 µm and more specifically less than 100 µm, or even less than 50 µm.

The D-amino-Acid Oxydase (DAAO) may be flavoenzyme that contains a molecule of covalently or non-covalently bound flavin adenine dinucleotide as cofactor, which is the site of redox reaction

More precisely, the means for oxidation of the D-amino acid may be at least one D-amino-acid oxidase (DAAO) which doesn't need the addition of a cofactor in the medium to oxidise the D-amino-acid. The DAAO may have a natural or artificial cofactor that is tightly bound to the apoprotein moiety (i.e., no inactive apoprotein is produced under the assay conditions), and/or a high specific activity.
The D-Amino-Acid Oxidase may have a high specific activity, which can be of at least 20, in particular at least 40, or even at least 55 units/mg for at least one, or only one, specific D-amino acid. More precisely, the D-Amino-Acid Oxidase may have a high specific activity, which can be of at least 20, in particular at least 40, or even at least 55 units/mg D-serine and/or least 50, in particular at least 75, or even at least 100 units/mg for D-alanine. One DAAO unit is defined as the amount of enzyme that converts 1 µmole of D-alanine per minute at 25 °C.

By "derivatives" is meant in the instant invention, an enzyme having at least 65 %, in particular at least 75 %, more particularly at least 85 %, notably at least 90 %, even at least 95 %, even more particularly at least 99 % identity of the amino acids with the corresponding enzyme. The derivatives have the same type of activity, for example a D-amino acid oxidase, in particular with an efficiency of the same order, i.e. at least 50 %, more specially at least 75 %, in particular at least 100 %, even more than 150 % of the efficiency of the original enzyme. The efficiency of the enzyme may be defined as the ratio k_{cat}/kₘ, assayed with an oxygen electrode at pH 8.5 and 25 °C, at air oxygen saturation ([O₂] = 0.253 mM) [G. Molla, C. Vegezzi, M.S. Pilone, L. Pollegioni, Overexpression in Escherichia coli of a recombinant chimeric Rhodotorula gracilis D-amino acid oxidase, Prot. Express. Purif., 14 (1998) 289-294].

D-Serine is a substrate of the D-Amino-Acid Oxidase (DAAO). The D-Serine may be a natural or a synthetic.

The means for oxidation of the D-amino-acid may be immobilized on the electrode by:
- Covalent immobilization, for example by using bifunctional agents, such as glutaraldehyde,
   - by using vapors of the bifunctional agent, or
   - by preliminary mixing solution of bifunctional agents with enzymatic mixture
- Entrapping D-Amino-Acid Oxidase (DAAO), for example into a reticular matrix, which can be formed by different types of polymeric reactions, in particular stimulated by UV, irradiation or chemical catalyst, or electrogenerated, like polymers of phenylenediamine, pyrrole or aniline,
- Immobilisation into the body of the electrode, such as paste type electrode, for example mix of enzyme, graphite powder, mediator, and/or
- Adsorption, in particular on a high surface layered material, such as cellulose acetate, nylon, inorganic gels (for example silica gel) and such types of membranes.

The means for optimising the detection of compound B may correspond to means for the catalysis of hydrogen peroxide oxidation.
The means for optimising the detection of said hydrogen peroxide may be chosen from:
- an electrochemical pre-treatment of the electrode, in particular carbon based electrode, and
- deposition, for example via electrochemical or vacuum deposition, of one or several metals, such as metals catalysing the oxidation of hydrogen peroxide,
- deposition of an artificial system catalysing the oxidation of compound B, such as artificial peroxydase system, in particular based on Prussian Blue,
- Horseradish peroxidase (HRP), and
- use of nanoparticles, for example of Gold, Platinum, Silicon oxides, Zinc, carbon nanotubes, etc.

When the means for optimising the detection of said hydrogen peroxide is a metal it can be chosen from the noble metal group comprising platinum, gold, ruthenium, rhodium, palladium, iridium, osmium, and from other metals like iron, chromium, nickel and tungsten.

The means for reducing interferences may be limiting oxidation of compounds, other than hydrogen peroxide, which may be oxidised by the electrode, in particular by limiting the access and/or the contact of these compounds with the electrode.

The means for reducing interferences may be chosen from:
- electrogenerated polymeric membranes, for example based on monomers like benzene and its derivatives, such as phenylenediamine, resorcinol, phenol; naphthalene and its derivatives; pyrrole and its derivatives; aniline and its derivatives; and combinations thereof,
- membranes deposited from solutions of polymers, charged, like Nafion, or neutral, like polyurethane and polyvinyl chloride, or derivatives of cellulose,
- non polymeric charged molecule layer, for example such a layer is deposited via solution, such as phospholipids and fatty acids, like stearic acid,
- use of at least an enzyme that degrades interfering molecule(s), for example ascorbic acid oxidase, in particular this enzyme may be immobilized on the electrode, and
- use of electrochemical methods allowing to discriminate between different molecules, for example pulsed amperometry, cyclic voltammetry or pulsed voltammetry.
The means for reducing interferences may be a layer of a compound chosen from the group comprising, poly-meta-, para- or ortho- phenylenediamine (PPD), substituted naphthalene based polymers, rejecting membrane or a rejecting polymer membrane.

Among the electrode materials that can be used the following may be cited:
- electrodes based on platinum, rhodium, palladium, gold, ruthenium, possibly under an alloy form, for example with gold, iridium or other noble metals, among such alloys an example is platinum 90 % and iridium 10 %
- electrodes based on carbon materials, graphite (rods), glassy carbon (rods), carbon fibres, diamond,
- electrodes covered by nanoparticles of noble metals, carbon materials or a mixture thereof, and
- ceramic based amperometric electrodes.
Some type of electrode materials may be excluded as they possibly lead to inactivation of the D-Amino-Acid Oxidase (DAAO), in particular Ag and Hg electrodes.

The electrode shape may be a disk, a ring, a rod, a cylinder, a cone or a hemisphere.

Following a specific embodiment, the microelectrode comprises, or consists of, a carbon fibre with the working part covered by a layer of ruthenium, a layer of PPD and a layer of DAAO.

Following another aspect, the subject matter of the invention is a device for detecting and/or measuring the concentration of a D-Serine in a medium, in particular in vivo, comprising an electrode as defined above.

This device may further comprise an acquisition card driving an amplifier, in particular equipped with a two or three electrodes potentiostat.

This device may also comprise a working electrode and a reference electrode for the two-electrode system, and an additional auxiliary or counter electrode for the three-electrode system.

Following another aspect, the subject matter of the invention is a method for detecting and/or measuring the concentration of a D-Serine in a medium, in particular in vivo, comprising the following steps:
- a working microelectrode according to any of claims 1 to 9 is placed in the medium to study,
- a working potential is applied, in particular by cyclic voltammetry, pulsed voltammetry/amperometry or by applying a constant potential.

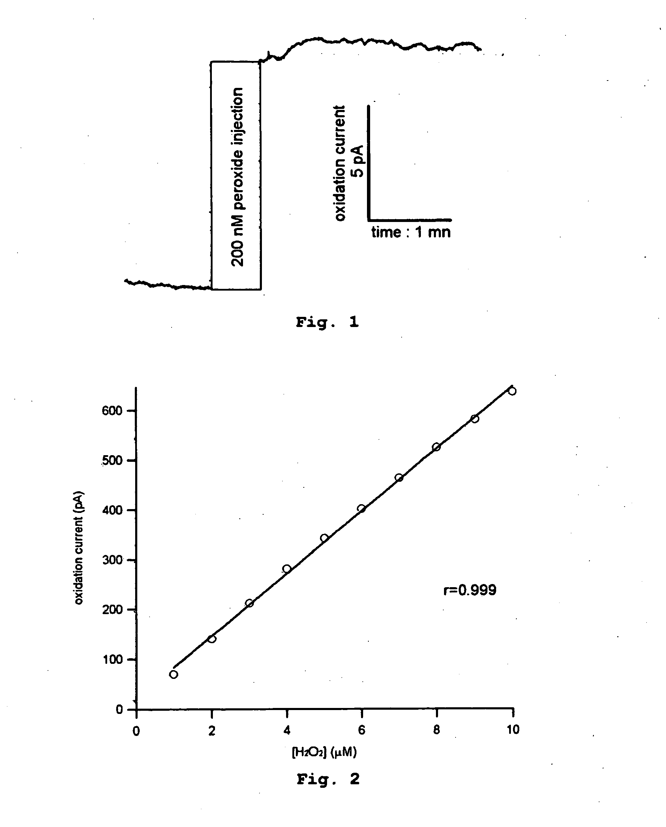

The potential may be fixed between -1 and +1 V vs. Ag/AgCl for the detection of H₂O₂.
An efficient detection method is the continuous amperometry at a fixed potential of +0,55 V vs. Ag/AgCl. In this case the peroxide oxidation is seen via a jump of current at the level of working electrode (Fig. 1). The relationship between oxidation and peroxide concentration in the solution is linear, in particular in a range of 0,01 µM to 10 µM (Fig. 2).

Following another aspect, a subject matter of the invention is a method for making a microelectrode comprising the following steps:
- treating the electrode in order to increase its sensitivity to hydrogen peroxide, in particular via a metallization, for example an electrodeposition of a metal,
- the deposition of a polymer film in order to discriminate hydrogen peroxide from other compounds which may be oxidised by the electrode,
- the deposition of a film of an enzyme oxidising D-Serine to hydrogen peroxide on the electrode, said enzyme being a D-Serine oxydase from a yeast or a microorganism chosen from the group comprising *Rhodotorula gracilis DAAO (RgDAAO), Trigonopsis variabilis DAAO, V. luteoalbum DAAO, F. oxysporum DAAO,* and then immobilizing said enzyme.

The enzyme may be immobilised via a cross-linking process, for example with an aldehyde, such as glutaraldehyde, or with other molecules such as poly(ethylene glycol) 400 diglycidyl ester (PEGDGE) or may be imprisoned in the material of the electrode, such as the carbon paste. It can also be entrapped or encapsulated in a polymer film, such as polypyrrole.

The metal covering the working part of the electrode may be deposited via the classical methods or by a gel impregnated with the metal, such as the osmium hydrogel.

### Examples

### Example 1: Preparation of a microelectrode for measuring D-serine concentration

### 1. Preparation of the carbon fibre electrode

A 7 µm diameter carbon fibre electrode (Goodfellow Cambridge Ltd., Huntington, UK) glued to a copper wire by a silver paint (Radiospares, Beauvais, FR) deposition. The wire is placed in a glass micropipette on which the extremity is cut in order to allow the carbon fibre to protrude. The junction between glass micropipette and the carbon fibre is sealed by an epoxy resin, in order to insure its tightness, and the carbon fibre is cut at 150 µm.
The electrode is then put into ethanol for 20 minutes to clean the fibre of its impurities.

### 2. Electrodeposition of ruthenium

The electrodeposition of ruthenium has been done by applying for 20 minutes a potential of -450 mV Vs Ag/AgCl to the carbon fibre electrode which is in a solution of RuCl₃ at a concentration of 100 µg/ml and pH 2.5 (ruthenium atomic absorption standard solution, Sigma, Saint Quentin, France).

After the deposition of Ruthenium, the electrode has been cycled 15 times between 0 and 700 mV for stabilisation.

### 3. Deposition of a PPD film layer

The deposition has been done electrochemically by putting the electrode 20 minutes in a solution of 100 mM of m-PD and applying a potential of +700 mV vs. Ag/AgCl.

### 4. Deposition of a D-amino-acid oxidase (DAAO) film

A film of enzyme is deposited on the electrode by dipping the electrode into an enzyme solution comprising 56 mg/ml of RgDAAO, 25 mg/ml Bovine Serum Albumine (BSA) and 1 % glycerol. The electrode is removed from the solution and then the enzyme is immobilised by exposition to vapours of a 50 % glutaraldehyde solution.
Alternatively the enzyme may be immobilized by using a solution of enzyme comprising glutaraldehyde 0.17 % ; glycerol 2 % ; RgDAAO 37 mg/ml ; BSA 17 mg/ml.
The thickness of the DAAO film obtained is about 15 to 25 µm.

### Example 2: Device for measuring the concentration of D-serine

The system of command and acquisition is composed of an acquisition card ITC-18 (Instrutech Corporation, Greatneck, NY, USA), driving an amplifier VA-10 (NPI Electronics, Tamm, Germany) equipped with a two-electrode (working electrode corresponding to the microelectrode of the invention and home made reference electrode composed of a chlorided silver wire) potentiostat. The acquisition card is driven with the SVoltare software.

### Example 3: cell cultures.

Primary cultures of cortical astrocytes were prepared from newborn rats and cultured until reaching confluence (14 days). The astrocytes were then treated 5 days with 8 µM cytosine arabinofuranoside in order to eliminate remaining microglia.
The cells were incubated 30 minutes in 500 µM D-serine, and washed in Krebs buffer prior to the recording. The microelectrode of the invention has been set on top of the cells without touching them. To determine the specificity of the signal, a control electrode, coated with bovine serum albumine (BSA) instead of D-Amino-Acid Oxidase (DAAO), was set at ~100 µm from the first one. Mechanical stimulation of an astrocyte with a glass micropipette produced an increase in the oxidation current at the working electrode, reflecting a release of D-serine by the cells (the current at the control/BSA electrode was not affected by the stimulation).

### Example 4: in vivo recordings in the CNS of an anesthetized rat

Male Wistar rats weighing between 300 and 400g were anesthesized with chloral hydrate and placed in a stereotaxic apparatus. The reference electrode was laid on the surface of the skull and the working electrode was inserted into the frontal cortex at about 1 mm under the pial surface.

After a 30 min stabilization time, the oxidation current at the working electrode under a potential of 0,5V vs. Ag/AgCl was recorded.
D-serine was then injected intraperitoneally at a concentration of 1g/kg of body weight.
After a few minutes the signal by our method was increased by about 100-200 pA, suggesting that D-serine penetrating through the blood-brain barrier into the central nervous system was detected by the microbiosensor.

## Claims

1. Microelectrode, for measuring and/or detecting the concentration of a D-Serine, said microelectrode comprising:
- means for oxidation of said D-Serine thereby generating hydrogen peroxide, said means being a D-amino-acid oxydase from a yeast or a microorganism chosen from the group comprising *Rhodotorula gracilis DAAO (RgDAAO), Trigonopsis variabilis DAAO, V. luteoalbum DAAO, F. oxysporum DAAO,*
- means for optimising the detection of said hydrogen peroxide, said means being means for the catalysis of hydrogen peroxide oxidation, and
- means for reducing interferences, said means being limiting oxidation of compounds other than hydrogen peroxide.

2. Microelectrode according to claim 1 wherein the means for oxidation of the D-amino acid is a D-amino-acid oxidase (DAAO) which doesn't need the addition of a cofactor in the medium to oxidise the D-amino-acid and/or shows a high specific activity.

3. Microelectrode according claims 1 or 2, wherein the means for oxidation of the D-amino-acid is immobilized on the electrode by:
- Covalent immobilization,
- Entrapping D-Amino-Acid Oxidase (DAAO),
- Immobilisation into the body of the electrode, and/or
- Adsorption.

4. Microelectrode according to any of claims 1 to 3, wherein the means for optimising the detection of said hydrogen peroxide is chosen from:
- an electrochemical pre-treatment of the electrode, in particular carbon based electrode, and
- deposition, for example via electrochemical or vacuum deposition, of at least one metal, such as a metal catalysing the oxidation of hydrogen peroxide,
- deposition of an artificial system catalysing the oxidation of hydrogen peroxide, such as artificial peroxydase system, in particular based on Prussian Blue,
- Horse Radish peroxidase (HRP), and
- use of nanoparticles.

5. Microelectrode according to claim 4, wherein the metal is chosen from the noble metal group comprising platinum, gold, ruthenium, rhodium, palladium, iridium, osmium, and from other metals like iron, chromium, nickel, tungsten.

6. Microelectrode according to any of claims 1 to 5, wherein the means for reducing interferences are limiting oxidation of compounds, other than hydrogen peroxide, which may be oxidised by the electrode, in particular by limiting the access and/or the contact of these compounds with the electrode.

7. Microelectrode according to any of claims 1 to 6, wherein means for reducing interferences are chosen from:
- electrogenerated polymeric membranes,
- membranes deposited from solutions of polymers, charged, or neutral,
- non polymeric charged molecule layer,
- use of at least an enzyme that degrades interfering molecule(s), and
- use of electrochemical methods allowing to discriminate between different molecules.

8. Microelectrode according to any of claims 1 to 7, wherein the electrode is chosen from the group comprising:
- electrodes based on platinum, rhodium, palladium, gold, ruthenium
- electrodes based on carbon materials, graphite and glossy carbon, carbon fibres, diamond,
- electrodes covered by nanoparticles based on noble metals, carbon materials or a mixture thereof, and
- ceramic based amperometric electrodes.

9. Microelectrode according to any of claims 1 to 8, wherein it is a carbon fibre electrode covered by a layer of ruthenium, a layer of PPD and a layer of D-Amino-Acid Oxidase (DAAO).

10. Device for detecting and/or measuring the concentration of a D-Serine in a medium, in particular in vivo, comprising an electrode according to any of claims 1 to 9.

11. Method for detecting and/or measuring the concentration of a D-Serine in a medium comprising the following steps:
- a working microelectrode according to any of claims 1 to 9 is placed in the medium to study,
- a working potential is applied, in particular by cyclic voltammetry, pulsed voltammetry/amperometry or by applying a constant potential.

12. Method for making a microelectrode comprising the following steps:
- treating the electrode in order to increase its sensitivity to hydrogen peroxide, in particular via a metallization,
- the deposition of a polymer film in order to discriminate hydrogen peroxide from other compounds which may be oxidised by the electrode,
- the deposition of a film of an enzyme oxidising D-Serine to hydrogen peroxide on the electrode, said enzyme being a D-Serine oxydase from a yeast or a microorganism chosen from the group comprising *Rhodotorula gracilis DAAO (RgDAAO), Trigonopsis variabilis DAAO, V. luteoalbum DAAO, F. oxysporum DAAO*, and then immobilizing said enzyme.

## Patentansprüche

1. Mikroelektrode zum Messen und/oder Nachweis der Konzentration von D-Serin, wobei besagte Mikroelektrode folgendes enthält :
- Mittel zur Oxidation des besagten D-Serins, wodurch Wasserstoffperoxid gebildet wird, und besagtes Mittel eine D-Aminosäure-Oxidase aus einer Hefe oder einem Mikroorganismus ist, die aus der Gruppe gewählt werden, zu der *Rhodotorula gracilis DAAO (RgDAAO), Trigonopsis variabilis DAAO, V. luteoalbum DAAO,* F. *oxysporum DAAO gehören*,
- Mittel zum verbesserten Nachweis des besagten Wasserstoffperoxids, wobei besagte Mittel aus Mitteln zur Katalyse von Wasserstoffperoxid bestehen, und
- Mittel zur Reduzierung von Interferenzen, wobei besagte Mittel die Oxidierung von anderen Verbindungen ausser Wasserstoffperoxid begrenzen.

2. Mikroelektrode gemäss Anspruch 1, wobei das Mittel zur Oxidation der D-Aminosäure eine D-Aminosäure-Oxidase (DAAO) ist, die keine Zugabe eines Kofaktors in das Medium erfordert, um D-Aminosäure zu oxydieren und/oder die eine hohe spezifische Aktivität zeigt.

3. Mikroelektrode gemäss Anspruch 1 oder 2, wobei das Mittel zur Oxidation der D-Aminosäure auf der Elektrode folgendermassen immobilisiert wird:
- Kovalente Immobilisierung,
- Aufnahme der D-Aminosäure-Oxidase (DAAO),
- Immobilisierung im Elektrodenhalter und/oder
- Adsorption.

4. Mikroelektrode gemäss einem der Ansprüche 1 bis 3, wobei zur Verbesserung des Wasserstoffperoxidnachweises unter folgenden Mitteln gewählt wird :
- eine elektrochemische Vorbehandlung der Elektrode, insbesondere einer Elektrode auf Kohlenstoffbasis, und
- Ablagerung, zum Beispiel durch elektrochemische Ablagerung oder durch Vakuummetallisierung zumindest eines Metalls, wie eines Metalls, das die Oxidation von Wasserstoffperoxid katalysiert,
- Ablagerung eines künstlichen Systems, das die Oxidation von Wasserstoffperoxid katalysiert, wie eines künstlichen Peroxidasesystems, insbesondere auf Preussischblau-Basis,
- Peroxidase von Schwarzem Rettich (HRP), und
- Verwendung von Nanopartikeln.

5. Mikroelektrode gemäss Anspruch 4, in der das Metall aus der Edelmetallgruppe gewählt wird, die Platin, Gold, Ruthenium, Rhodium, Palladium, Iridium, Osmium umfasst, und aus anderen Metallen, wie Eisen, Chrom, Nickel, Wolfram.

6. Mikroelektrode gemäss einem der Ansprüche 1 bis 5, in der die Mittel zur Verringerung der Interferenzen Mittel zur Begrenzung der Oxidation von Verbindungen ausser Wasserstoffperoxid sind, die durch die Elektrode oxidiert werden können, insbesondere durch Begrenzung des Zugangs und/oder Kontakts dieser Verbindungen mit der Elektrode.

7. Mikroelektrode gemäss einem der Ansprüche 1 bis 6, wobei als Mittel zur Verringerung der Interferenzen unter folgenden Mitteln gewählt wird :
- elektrogenerierte Polymermembranen,
- Membranen, die aus elektrisch geladenen oder neutralen Polymerlösungen abgelagert werden,
- Schicht elektrisch geladener nicht polymerer Moleküle,
- Verwendung von mindestens einem das (die) interferierende(n) Molekül(e) abbauenden Enzyms, und
- Verwendung elektrochemischer Methoden, die eine Unterscheidung verschiedener Moleküle ermöglichen.

8. Mikroelektrode gemäss einem der Ansprüche 1 bis 7, wobei die Mikroelektrode unter einer der Gruppen gewählt wird, die folgendes enthalten :
- Elektroden auf Platin-, Rhodium-, Palladium-, Gold-, Rutheniumbasis
- Elektroden auf Basis von Kohlenstoffmaterialien, Graphit und glänzendem Kohlenstoff, Kohlenstofffasern, Diamant
- Elektroden, die von Nanopartikeln auf Edelmetall-, Kohlenstoffmaterialienbasis oder einer Mischung hieraus überzogen sind, und
- amperometrische Elektroden auf Keramikbasis.

9. Mikroelektrode gemäss einem der Ansprüche 1 bis 8, wobei die Kohlenstofffaserelektrode mit einer Schicht Ruthenium, einer Schicht PPD und einer Schicht D-Aminosäure-Oxidase (DAAO) überzogenen ist.

10. Vorrichtung zum Nachweis und/oder der Messung der D-Serin-Konzentration in einem Milieu, insbesondere *in vivo,* die eine Elektrode gemäss einem der Ansprüche 1 bis 9 enthält.

11. Verfahren zum Nachweis und/oder der Messung der D-Serin-Konzentration in einem Medium mit folgenden Schritten:
- eine Mikroelektrode zur Analyse gemäss einem der Ansprüche 1 bis 9 wird in dem zu analysierenden Medium angebracht,
- ein Messpotential wird angewandt, insbesondere mittels zyklischer Voltamperometrie, pulsierender Voltamperometrie/Amperometrie oder durch Anwendung eines konstanten Potentials.

12. Verfahren zur Fertigung einer Mikroelektrode mit folgenden Schritten :
- Behandlung der Elektrode zur Verstärkung ihrer Empfindlichkeit für Wasserstoffperoxid, insbesondere durch Metallisierung,
- Ablagerung eines Polymerfilms, so dass das Wasserstoffperoxid von anderen Verbindungen, die durch die Elektrode oxidiert werden können, unterschieden werden kann,
- Ablagerung eines Enzymfilms, der D-Serin in Wasserstoffperoxid auf der Elektrode oxidiert, wobei das besagte Enzym eine D-Serin-Oxydase aus einer Hefe oder einem Mikroorganismus ist, die aus der Gruppe gewählt werden, zu der *Rhodotorula gracilis DAAO (RgDAAO), Trigonopsis variabilis DAAO, V. luteoalbum DAAO, F. oxysporum* DAAO gehören, und dann Immobilisierung des besagten Enzyms.

## Revendications

1. Microélectrode, pour mesurer et/ou détecter la concentration en D-Sérine, ladite microélectrode comprenant :
- un moyen pour l'oxydation de ladite D-Sérine, générant ainsi du peroxyde d'hydrogène, ledit moyen étant un acide D-amino-acide-oxydase provenant d'une levure ou d'un microorganisme choisi dans le groupe comprenant *Rhodotorula gracilis DAAO (RgDAAO), Trigonopsis variabilis DAAO, V. luteoalbum DAAO, F. oxysporum DAAO,*
- un moyen pour optimiser la détection dudit peroxyde d'hydrogène, ledit moyen étant un moyen de catalyse de l'oxydation de peroxyde d'hydrogène, et
- des moyens pour réduire des interférences, lesdits moyens étant de limiter l'oxydation de composés autres que le peroxyde d'hydrogène.

2. Microélectrode selon la revendication 1 dans laquelle le moyen pour l'oxydation du D-amino-acide est une D-amino-acide-oxydase (DAAO) ne nécessitant pas l'addition d'un cofacteur dans le milieu pour oxyder le D-amino-acide et/ou montrant une activité spécifique élevée.

3. Microélectrode selon les revendication 1 ou 2, dans laquelle le moyen pour l'oxydation de la D-amino-acide est immobilisé sur une électrode par :
- immobilisation covalente,
- piégeage de la D-amino-acide-oxydase (DAAO),
- immobilisation à l'intérieur du corps de l'électrode, et/ou
- adsorption.

4. Microélectrode selon l'une quelconque des revendications 1 à 3, dans laquelle le moyen pour optimiser la détection dudit peroxyde d'hydrogène est choisi parmi :
- un prétraitement électrochimique de l'électrode, en particulier un électrode à base de carbone, et
- le dépôt, par exemple par voie électrochimique ou par dépôt sous vide, d'au moins un métal, tel qu'un métal catalysant l'oxydation du peroxyde d'hydrogène,
- le dépôt d'un système artificiel catalysant l'oxydation du peroxyde d'hydrogène, tel qu'un système peroxydase artificiel à base de bleu de Prusse,
- la peroxydase de radis noir (HRP), et
- l'utilisation de nanoparticules.

5. Microélectrode selon la revendication 4, dans laquelle le métal est choisi parmi le groupe des métaux nobles comprenant le platine, l'or, le ruthénium, le rhodium, le palladium, l'iridium, l'osmium, et parmi d'autres métaux comme le fer, le chrome, le nickel, le tungstène.

6. Microélectrode selon l'une quelconque des revendications 1 à 5, dans laquelle les moyens pour réduire des interférences limitent l'oxydation de composés, autres que le peroxyde d'hydrogène, qui peuvent être oxydés par l'électrode, en particulier en limitant l'accès et/ou le contact de ces composés avec l'électrode.

7. Microélectrode selon l'une quelconque des revendications 1 à 6, dans laquelle les moyens pour réduire des interférences sont choisis parmi:
- des membranes polymériques électrogénérées,
- des membranes déposées à partir de solution de polymères chargés, ou neutres,
- une couche de molécules chargées non polymériques,
- l'utilisation d'au moins une enzyme dégradant une(des) molécule(s) interférente(s), et
- l'utilisation de méthodes électrochimiques permettant de distinguer les différentes molécules.

8. Microélectrode selon l'une quelconque des revendications 1 à 7, dans laquelle l'électrode est choisie dans le groupe comprenant :
- les électrodes à base de platine, rhodium, palladium, or ruthénium,
- les électrodes à base de matériaux carbonés, de graphite, et de carbone brillant, de fibres de carbones, de diamant,
- les électrodes couvertes par des nanoparticules à base de métaux nobles, de matériaux carbonés ou un mélange de ceux-ci, et
- les électrode ampérométriques à base de céramique.

9. Microélectrode selon l'une quelconque des revendications 1 à 8, dans laquelle elle est une électrode en fibre de carbone recouverte par une couche de ruthénium, une couche de PPD et une couche de D-amino-acide-oxydase (DAAO).

10. Dispositif pour détecter et/ou mesurer la concentration en D-Sérine dans un milieu, en particulier *in vivo,* comprenant une électrode selon lune quelconque des revendications 1 à 9.

11. Procédé pour détecter et/ou mesurer la concentration en D-Sérine dans un milieu comprenant les étapes suivantes :
- une microélectrode à étudier selon l'une quelconque des revendications 1 à 9 est placé dans le milieu à étudier,
- un potentiel à étudier est appliqué, en particulier par un voltampérométie cyclique, voltampérométrie/ampérométrie pulsée ou par application d'un potentiel constant.

12. Procédé de fabrication d'une microélectrode comprenant les étapes suivantes :
- traitement de l'électrode de manière à augmenter sa sensibilité a peroxyde d'hydrogène, en particulier par une métallisation,
- dépôt d'un film polymère de manière à distinguer le peroxyde d'hydrogène des autres composés qui peuvent être oxydés par l'électrode,
- dépôt d'un film d'enzyme oxydant la D-Sérine en peroxyde d'hydrogène sur l'électrode, ladite enzyme étant une D-Sérine oxydase provenant d'une levure ou d'un microorganisme choisi dans le groupe comprenant *Rhodotorula gracilis DAAO (RgDAAO), Trigonopsis variabilis DAAO, V. luteoalbum DAAO, F. oxysporum DAAO*, et puis immobilisation de ladite enzyme.
